(19) Europäisches Patentamt
European Patent Office
Office européen des brevets

(11) Veröffentlichungsnummer: **0 305 845 B1**

(12) # EUROPÄISCHE PATENTSCHRIFT

(45) Veröffentlichungstag der Patentschrift: **28.10.92**

(51) Int. Cl.[5]: **C07D 213/61**, A61K 31/44, A23K 1/16

(21) Anmeldenummer: **88113612.1**

(22) Anmeldetag: **22.08.88**

Die Akte enthält technische Angaben, die nach dem Eingang der Anmeldung eingereicht wurden und die nicht in dieser Patentschrift enthalten sind.

(54) **Pyridylethanolamine, diese Verbindungen enthaltende Arzneimittel und Verfahren zu ihrer Herstellung.**

(30) Priorität: **02.09.87 DE 3729284**

(43) Veröffentlichungstag der Anmeldung: **08.03.89 Patentblatt 89/10**

(45) Bekanntmachung des Hinweises auf die Patenterteilung: **28.10.92 Patentblatt 92/44**

(84) Benannte Vertragsstaaten: **AT BE CH DE ES FR GB GR IT LI LU NL SE**

(56) Entgegenhaltungen:
**EP-A- 0 254 856**
**EP-A- 0 256 420**

(73) Patentinhaber: **Dr. Karl Thomae GmbH**
**Postfach 1755**
**W-7950 Biberach 1(DE)**

(72) Erfinder: **Reiffen, Manfred, Dr. Dipl.-Chem.**
**Matthias-Erzberger-Strasse 40**
**W-7950 Biberach 1(DE)**
Erfinder: **Grell, Wolfgang, Dr. Dipl.-Chem.**
**Geschwister-Scholl-Strasse 18**
**W-7950 Biberach 1(DE)**
Erfinder: **Sauter, Robert, Dr. Dipl.-Chem.**
**Albert-Schweitzer-Weg 9**
**W-7958 Laupheim(DE)**
Erfinder: **Hurnaus, Rudolf, Dr. Dipl.-Chem.**
**Silcherstrasse 19**
**W-7950 Biberach 1(DE)**
Erfinder: **Rupprecht, Eckhard, Dr. Dipl.-Biol.**
**Riedbachstrasse 15**
**W-7960 Aulendorf-Tannhausen(DE)**
Erfinder: **Mark, Michael, Dr.**
**Schlehenhang 17**
**W-7950 Biberach 1(DE)**

EP 0 305 845 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

In der nicht vorveröffentlichten EP-A-0,254,856 werden bereits Pyridylethanolamine, die eine katabolische Wirkung aufweisen, und in der EP-A-0,256,420 ebenfalls Pyridylethanolamine beschrieben, die eine leistungsfördernde Wirkung aufweisen.

Es wurde nun gefunden, daß die in den vorstehend erwähnten Offenlegungsschriften nicht expressis verbis vorbeschriebenen Pyridylethanolamine der Formel

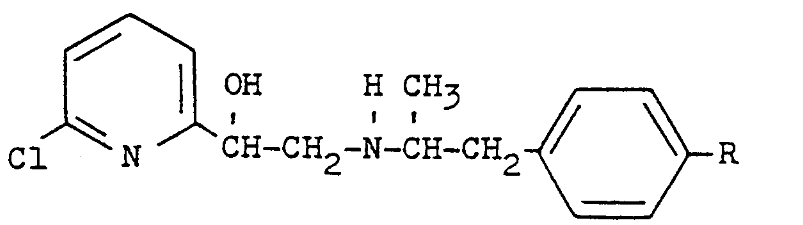

,(I)

in der
R eine Carboxymethoxy-, Carbomethoxymethoxy- oder 2-Hydroxyethoxygruppe darstellt, deren optische Isomere und Diastereomere sowie deren Säureadditionssalze, insbesondere für die pharmazeutische Anwendung deren physiologisch verträglichen Säureadditionssalze mit anorganischen oder organischen Säuren, wertvolle pharmakologische Eigenschaften aufweisen, nämlich eine Wirkung auf den Stoffwechsel, vorzugsweise eine blutzuckersenkende und körperfettreduzierende Wirkung, sowie eine senkende Wirkung auf die atherogenen Lipoproteine VLDL und LDL.

Gegenstand der vorliegenden Erfindung sind somit die Pyridylethanolamine der obigen allgemeinen Formel I, deren Säureadditionssalze, diese Verbindungen enthaltende Arzneimittel, deren Verwendung und Verfahren zu ihrer Herstellung.

Erfindungsgemäß erhält man die neuen Verbindungen nach folgenden Verfahren:

a) Umsetzung einer Verbindung der Formel

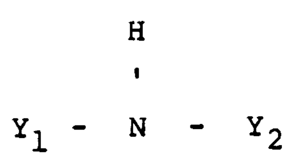

,(II)

in der
$Y_1$ eine Gruppe der Formel

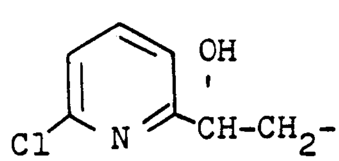

und
$Y_2$ eine Gruppe der Formel

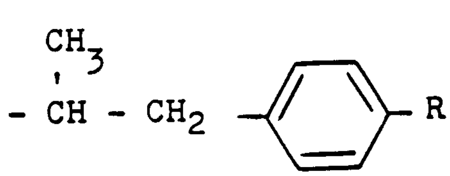

darstellt, in der
R wie eingangs definiert ist, wobei jedoch einer der Reste
$Y_1$ oder $Y_2$ ein Wasserstoffatom darstellen muß, mit einer Verbindung der Formel

$Z_1$ - U ,(III)

in der
U eine Gruppe der Formeln

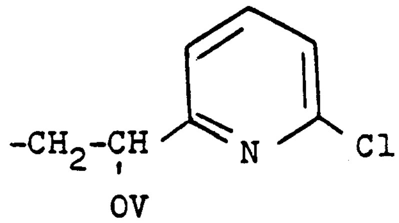

oder

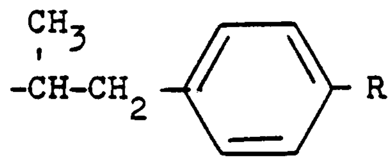

darstellt, wobei
R wie eingangs definiert ist,
$Z_1$ und V zusammen eine Bindung oder
$Z_1$ eine nukleophile Austrittsgruppe wie ein Halogenatom,
z.B. ein Chlor-, Brom- oder Jodatom, oder eine Sulfonyloxygruppe, z.B. eine Methansulfonyloxy-, p-Toluolsulfonyloxy-oder Ethoxysulfonyloxygruppe, und
V ein Wasserstoffatom bedeuten.

Die Umsetzung wird zweckmäßigerweise in einem Lösungsmittel oder Lösungsmittelgemisch wie Methanol, Ethanol, Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuß der eingesetzten Verbindung der allgemeinen Formel III und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin, N,N-Diisopropylethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150 °C, vorzugsweise bei Temperaturen zwischen 50 und 120 °C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch mit einem entsprechenden Epoxid der Formel III ohne Zusatz eines säurebindenden Mittels durchgeführt.

b) Reduktive Aminierung einer Carbonylverbindung der Formel

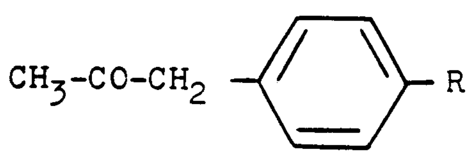

,(IV)

in der
R wie eingangs definiert ist, mit einem Amin der Formel

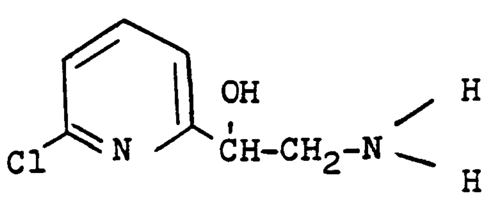

,(V)

Die reduktive Aminierung wird in einem geeigneten Lösungsmittel wie Methanol, Ethanol, Tetrahydrofuran, Dioxan oder Acetonitril in Gegenwart eines geeigneten Reduktionsmittels wie einem geeigneten komplexen Metallhydrid, vorzugsweise jedoch in Gegenwart von Natriumcyanborhydrid bei einem pH-Wert von 5 bis 7, bei Temperaturen zwischen 0 und 50 °C, vorzugsweise jedoch bei Raumtemperatur, durchgeführt.

c) Reduktion einer Verbindung der Formel

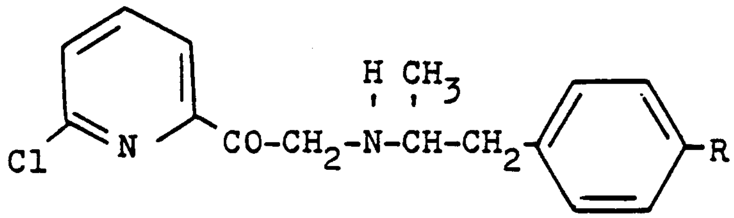
,(VI)

in der

R wie eingangs definiert ist.

Die Reduktion wird vorzugsweise in einem geeigneten Lösungsmittel wie Methanol, Ethanol, Diethylether oder Tetrahydrofuran in Gegenwart eines Metallhydrids wie Natriumborhydrid, Lithiumaluminiumhydrid, Diboran, Boran/Dimethylsulfid oder Natriumcyanborhydrid, vorzugsweise jedoch mit Natriumborhydrid in Methanol oder Ethanol, zwischen 0 und 40 °C, vorzugsweise jedoch bei Raumtemperatur, durchgeführt.

Bei der Reduktion mit einem komplexen Metallhydrid wie Lithiumaluminiumhydrid, Diboran oder Boran/Dimethylsulfid, kann eine im Rest R vorhandene Carbonylfunktion zu einer Methylengruppe mitreduziert werden.

d) Umsetzung einer Verbindung der Formel

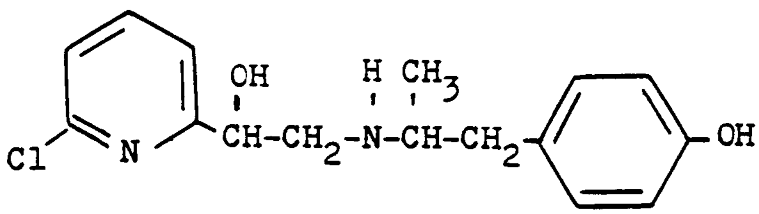
,(VII)

mit einer Verbindung der Formel

$Z_2$ - $R_1$ ,(VIII)

in der

$R_1$ eine Carboxymethyl-, Methoxycarbonylmethyl-, Ethoxycarbonylmethyl- oder 2-Hydroxy-ethylgruppe und

$Z_2$ eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, oder

$Z_2$ zusammen mit einem $\beta$-Wasserstoffatom des Restes $R_1$ ein Sauerstoffatom darstellen.

Die Umsetzung wird zweckmäßigerweise in einem Lösungsmittel wie Diethylether, Tetrahydrofuran, Dioxan, Methanol, Ethanol oder Dimethylformamid und vorzugsweise in Gegenwart eines säurebindenden Mittels wie Natriumhydroxid oder Kalium-tert.butylat, vorzugsweise jedoch in Gegenwart von Kaliumcarbonat oder Natriumhydrid, oder Pyridin, wobei eine organische Base wie Pyridin auch als Lösungsmittel dienen kann, oder zur Herstellung von 2-Hydroxy-ethoxy-Verbindungen der allgemeinen Formel I mit Ethylenoxid bei Temperaturen zwischen 0 und 100 °C, vorzugsweise jedoch bei Temperaturen zwischen 20 und 80 °C, durchgeführt.

Bei den Umsetzungen a) bis d) können reaktionsfähige Gruppen während der Umsetzung durch übliche Schutzreste geschützt werden, diese werden nach der Umsetzung durch übliche Verfahren wieder abgespalten.

Als Schutzreste für eine Carboxygruppe kommen beispielsweise die Benzyl-, tert.Butyl-, Tetrahydropyranyl-, Trimethylsilyl-, Benzyloxymethyl-, 2-Chlorethyl- oder Methoxymethylgruppe oder eine Phenacylgruppe wie die Benzoylmethylgruppe,
als Schutzreste für eine Amino- oder Alkylaminogruppe die Acetyl-, Benzoyl-, tert.Butoxycarbonyl-, Benzyloxycarbonyl-, Ethoxycarbonyl- oder Benzylgruppe und
als Schutzreste für eine Hydroxygruppe beispielsweise die Trityl-, Fluorenylmethyloxycarbonyl-, Benzyloxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzyl- oder Benzyloxycarbonylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

Erhält man erfindungsgemäß eine Verbindung der Formel I, in der R eine Methoxycarbonylmethoxygruppe darstellt, so kann diese mittels Hydrolyse in eine entsprechende Verbindung der Formel I, in der R eine Carboxymethoxygruppe darstellt, übergeführt werden.

Die nachträgliche Hydrolyse wird entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Methanol, Äthanol, Äthanol/Wasser, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches, durchgeführt.

Wie bereits eingangs erwähnt, können die neuen Verbindungen in Form ihrer Enantiomeren, Enantiomerengemische oder Racemate, oder auch in Form ihrer Diastereomerenpaare bzw. Diastereomerenpaargemische vorliegen.

So lassen sich die erhaltenen Verbindungen der Formel I auf Grund ihrer physikalischen-chemischen Unterschiede nach an sich bekannten Methoden, z.B. durch Chromatographie und/oder fraktionierte Kristallisation, in ihre Diastereomeren auftrennen. Ein so erhaltenes Enantiomerenpaar läßt sich anschließend nach an sich bekannten Methoden (siehe Allinger N. L. und Eliel E. L. in "Topics in Stereochemistry", Vol. 6, Wiley Interscience, 1971) in ihre optischen Antipoden auftrennen, z.B. durch Umkristallisieren aus einem optisch aktiven Lösungsmittel oder durch Umsetzen mit einer, mit der racemischen Verbindung Salze bildenden optisch aktiven Substanz, insbesondere Säuren, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Säuren sind z.B. die D- und L-Formen von Weinsäure, Di-o-Toluolweinsäure, Äpfelsäure, Mandelsäure, Camphersulfonsäure, Glutaminsäure, Asparaginsäure oder Chinasäure.

Die als Ausgangsstoffe verwendeten Verbindungen, welche selbstverständlich auch in ihren optisch reinen Formen verwendet werden können, erhält man nach literaturbekannten Verfahren (siehe "Thiazole and its Derivatives" in Heterocyclic Compounds, Vol. 34, und Advances in Heterocyclic Chemistry, Vol 17, ab S. 100 (1974)) bzw. sind literaturbekannt. Diese liegen teilweise nur im Reaktionsgemisch vor und können daher teilweise nicht isoliert werden.

Eine als Ausgangsstoff verwendete Verbindung der Formel II, V, VI oder VII erhält man durch entsprechende Alkylierung eines entsprechenden Amins. Diese sind teilweise Gegenstand der EP-A-0.239.815.

Eine als Ausgangsstoff verwendete Verbindungen der Formel III, in der $Z_1$ eine nukleophile Austrittsgruppe darstellt, erhält man durch Reduktion einer entsprechenden Acetylverbindung, z.B. mit einem komplexen Metallhydrid, oder durch Überführung einer entsprechenden Hydroxyverbindung in ihr reaktives Derivat.

Ein als Ausgangsstoff verwendetes Epoxid der Formel III erhält man beispielsweise durch Umsetzung eines entsprechenden Brom- oder Tosylethanols mit wässriger Kali- oder Natronlauge.

Desweiteren können die erhaltenen Verbindungen der Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Wie bereits eingangs erwähnt, weisen die neuen Verbindungen der Formel I, deren Enantiomeren, Enantiomerengemische oder Racemate, oder sofern sie mindestens 2 asymmetrische Kohlenstoffatome enthalten, auch deren Diastereomeren bzw. Diastereomerengemische und deren Säureadditionssalze, insbesondere für die pharmazeutische Anwendung deren physiologisch verträgliche Säureadditionssalze, wertvolle pharmakologische Eigenschaften auf, neben einer Hemmwirkung auf die Plättchenaggregation insbesondere eine Wirkung auf den Stoffwechsel, vorzugsweise eine blutzuckersenkende und körperfettreduzierende Wirkung, sowie eine senkende Wirkung auf die atherogenen $\beta$-Lipoproteine VLDL und LDL.

Die biologischen Eigenschaften der neuen Verbindungen wurden wie folgt untersucht:

1. Antidiabetische Wirkung:

Die antidiabetische Wirkung der erfindungsgemäßen Verbindungen läßt sich als eine blutzuckersenkende Wirkung bei Versuchstieren messen. Die zu untersuchenden Substanzen wurden dazu in 1,5%iger Methylzellulose suspendiert und weiblichen Mäusen eigener Zucht mittels Schlundsonde appliziert. 30 Minuten später wurde 1 g Glukose pro kg Körpergewicht in Wasser gelöst und subkutan appliziert. Weitere 30 Minuten später wurde aus dem retroorbitalen Venenplexus Blut entnommen. Aus dem Serum wurde Glukose mit der Hexokinase-Methode mit Hilfe eines Analysenphotometers bestimmt.

In der nachstehenden Tabelle sind die bei dieser Versuchsanordnung beobachteten Blutzuckersenkungen in % einer mitgeführten Kontrollgruppe zusammengestellt. Die statistische Auswertung erfolgt nach dem t-Test nach Student mit p = 0,05 als Signifikanzgrenze.

| Verbindung (Beispiel Nr.) | % Änderung vom Wert der Kontrollgruppe Dosis [mg/kg] | | |
|---|---|---|---|
| | 0,1 | 0,3 | 1,0 |
| 1 | | | -66 |
| 2 | -64 | | |

2. Antiadipöse Wirkung:

Die antiadipöse Wirkung der erfindungsgemäßen Verbindungen wurde in zwei Versuchsanordnungen nachgewiesen:

a) In der ersten Versuchsanordnung wurde die Steigerung der Lipolyse am Anstieg des Glyzerins im Serum gemessen. Der Versuchsablauf ist identisch mit der zur Testung auf blutzuckersenkende Wirkung vorstehend beschriebenen Versuchsan-Ordnung. Glyzerin wurde in einem kombinierten enzymatischkolorimetrischen Test mit Hilfe eines Analysenphotometers bestimmt. Die Ergebnisse sind in der nachfolgenden Tabelle in % einer mitgeführten Kontrollgruppe aufgeführt.

| Verbindung (Beispiel NR.) | % Änderung vom Wert der Kontrollgruppe Dosis [mg/kg] | | |
|---|---|---|---|
| | 0,1 | 0,3 | 1,0 |
| 1 | | | + 882 |
| 2 | + 454 | | |
| 3 | | + 309 | |

b) In der zweiten Versuchsanordnung zur Erfassung antiadipöser Wirkung der erfindungsgemäßen Verbindungen wurde die Abnahme des Fettgewebes am Beispiel des Ovarfettgewebes gemessen. Die Verbindungen wurden zu diesem Zwecke Mäusen einmal täglich mittels Schlundsonde in 1,5%iger Methylzellulosesuspension appliziert. Am fünften Tag wurde das Ovarfettgewebe herauspräpariert und gewogen.

3. Kardiale Nebenwirkungen:

Das Auftreten von unerwünschten Nebenwirkungen auf das Herz konnte für die erfindungsgemäßen

Verbindungen für den therapeutisch angestrebten stoffwechselaktiven Dosisbereich ausgeschlossen werden. Als Nachweis diente die Messung der Herzfrequenz an Mäusen während der Testung auf blutzuckersenkende Wirkung (s.o.). Eine Stunde nach der oralen Applikation der Verbindungen wurde die Herzfrequenz mittels EKG-getriggertem Tachographen bestimmt. Die nachfolgende Tabelle gibt die Änderung der Herzfrequenz in % der Kontrollgruppe wieder.

| Verbindung (Beispiel Nr.) | % Änderung vom Wert der Kontrollgruppe | Dosis |
|---|---|---|
| 1 | +35 | 10 mg/kg |
| 2 | (+6,9) | 0,3 mg/kg |
| ( ) = nicht signifikant ($p > 0.05$) | | |

Desweiteren konnten bei den vorstehend beschriebenen Untersuchungen der erfindungsgemäßen Substanzen bei den applizierten Dosen keine toxischen Nebenwirkungen beobachtet werden. Diese sind daher gut verträglich.

Auf Grund ihrer pharmakologischen Eigenschaften eignen sich daher die neuen Verbindungen der Formel I, sowie deren physiologisch vertägliche Säureadditionssalze mit anorganischen oder organischen Säuren zur Behandlung sowohl des Diabetes mellitus als auch der Adipositas, insbesondere also zur Behandlung des adipösen Diabetikers. Außerdem können die neuen Verbindungen zur Prophylaxe und zur Behandlung atherosklerotischer Veränderungen der Gefäße, die vor allem bei Diabetikern und Obesen gehäuft auftreten, verwendet werden. Hierbei kann die erforderliche Dosis ganz auf den stoffwechselphysiologischen Bedarf des einzelnen Patienten abgestimmt werden, da diese über einen großen Dosisbereich frei von einer Herz/Kreislaufwirkung sind. Beim Erwachsenen liegen daher die Tagesdosis zwischen 1 und 3000 mg, vorzugsweise jedoch 1 bis 1000 mg, verteilt auf 1 bis 4 Dosen pro Tag. Hierzu lassen sich die obenerwähnten Verbindungen, gegebenenfalls in Kombination mit anderen Wirksubstanzen, in die üblichen galenischen Zubereitungsformen wie Pulver, Tabletten, Dragées, Kapseln, Suppositorien oder Suspensionen einarbeiten.

Desweiteren können die vorstehend erwähnten Verbindungen zur Behandlung fettsüchtiger Tiere wie Hunde und als Folge ihrer körperfettreduzierenden (lipolytischen) Wirkung zur Reduktion unerwünschter Fetteinlagerungen bei der Mastzucht, also zur Verbesserung der Fleischqualität von Masttieren wie Schweinen, Rindern, Schafen und Geflügel eingesetzt werden. Bei den Tieren kann die Applikation der oben genannten Verbindungen oral oder auch nicht-oral, z.B. als Futterzusatz oder durch Injektion oder auch über implantierte Minipumpen erfolgen. Die Tagesdosis liegt hierbei zwischen 0,01 und 100 mg/kg, vorzugsweise jedoch zwischen 0,1 bis 10 mg/kg Körpergewicht.

Die nachfolgenden Beispiele sollen die Erfindung näher erläutern:

## Beispiel 1

### N-[2-(4-Carbomethoxymethoxyphenyl)-1-methylethyl]-2-hydroxy-2-(6-chlor-pyridin-2-yl)ethanamin-hydrochlorid

0,23 g (0,00133 Mol) 2-Hydroxy-2-(6-chlor-pyridin-2-yl)ethanamin und 0,3 g (0,00133 Mol) 1-(4-Carbomethoxymethoxyphenyl)propan-2-on werden in 15 ml absolutem Methanol gelöst, mit 0,08 g (0,00133 Mol) Eisessig und 0,084 g (0,00133 Mol) Natriumcyanborhydrid versetzt und 16 Stunden bei Raumtemperatur gerührt. Anschließend wird auf Eis gegossen und mit Salzsäure angesäuert. Nach 5 Minuten wird bei 0°C bis 5°C mit Ammoniak alkalisch gestellt und mit Methylenchlorid extrahiert. Der Extrakt wird über Natriumsulfat getrocknet, eingedampft und über eine Kieselgelsäule mit Essigester/methanol = 9:1 als Eluens gereinigt. Die Base wird mit etherischer Salzsäure in das Hydrochlorid überführt.
Ausbeute: 0,276 g (46 % der Theorie),
Schmelzpunkt: ab 70°C (Zers.).

| | | | | |
|---|---|---|---|---|
| Ber.: | C 54,94 | H 5,82 | N 6,74 | Cl 17,07 |
| Gef.: | 54,60 | 6,00 | 7,00 | 17,40 |

Laut $^1$H-NMR-Spektrum (400 MHz, DMSO/$CD_3OD$) liegt ein ca. 1:1-Gemisch der Diastereomerenpaare vor.

δ = 1,17 ppm (d, >CH-$CH_3$)
δ = 1,20 ppm (d, >CH-$\underline{\overline{CH}}_3$)

Beispiel 2

N-[2-(4-(2-Hydroxyethoxy)phenyl)-1-methylethyl]-2-hydroxy-2-(6-chlor-pyridin-2-yl)ethanamin-hydrochlorid

Hergestellt analog Beispiel 1 durch Umsetzung von 2-Hydroxy-2-(6-chlor-pyridin-2-yl)ethanamin mit 1-(4-(2-Hydroxyethoxy)phenyl)propan-2-on und Natriumcyanborhydrid und anschließender Reinigung über eine Kieselgelsäule mit Essigester/Methanol = 9:1 als Eluens. Die Base wird mit etherischer Salzsäure in das Hydrochlorid überführt.
Ausbeute: 65 % der Theorie,
Schmelzpunkt: 68 ° C (Zers.)

| | | | | |
|---|---|---|---|---|
| Ber.: | C 55,81 | H 6,24 | N 7,23 | Cl 18,30 |
| Gef.: | 55,60 | 6,46 | 7,09 | 18,47 |

Laut $^1$H-NMR-Spektrum (400 MHz, d6-DMSO/$CD_3OD$) liegt ein ca. 1:1-Gemisch der Diastereomerenpaare vor:
δ = 1,121 ppm (d, >CH-$CH_3$)
δ = 1,136 ppm (d, >CH-$\underline{\overline{CH}}_3$)

Beispiel 3

N-[2-(4-Carboxymethoxyphenyl)-1-methylethyl]-2-hydroxy-2-(6-chlor-pyridin-2-yl)ethanamin

3,6 g (0,0095 Mol) N-[2-(4-Carbomethoxymethoxyphenyl)-1-methylethyl]-2-hydroxy-2-(6-chlor-pyridin-2-yl)ethanamin werden in 20 ml Methanol gelöst und 50 ml (0,05 Mol) 1 n Natronlauge zugetropft. Die Lösung wird für 30 Minuten bei Raumtemperatur gerührt und dann mit 50 ml 1 n Salzsäure (0,05 Mol) neutralisiert. Das Lösungsmittel wird im Vakuum abdestilliert und der Rückstand mit 20 ml Wasser und 20 ml Methanol über 16 Stunden verrührt, abgesaugt und mit wenig Methanol gewaschen.
Ausbeute: 1,8 g (52 % der Theorie),
Schmelzpunkt: 218 ° C (Zers.).

| | | | |
|---|---|---|---|
| Ber.: | C 59,25 | H 5,80 | N 7,68 |
| Gef.: | 59,47 | 5,83 | 7,55 |

Laut $^1$H-NMR-Spektrum (400 MHz, D6-DMSO/$CD_3OD$) liegt ein ca. 3:2-Gemisch der Diastereomerenpaare vor.
δ = 4,92 ppm (d, >CH-$CH_3$)
δ = 4,98 ppm (d, >CH-$\underline{\overline{CH}}_3$)

Beispiel 4

N-[2-(4-Carboxymethoxyphenyl)-1-methylethyl]-2-hydroxy-2-(6-chlor-pyridin-2-yl)ethanamin

Diastereomerenpaar A:

9,3 g eines 3:2-Gemisches der Diastereomerenpaare A und B werden in einem Gemisch von 600 ml Methanol und 900 ml Wasser heiß gelöst und durch Abkühlen im Eisbad zur Kristallisation gebracht. Dieser Umkristallisationsvorgang wurde noch dreimal mit 360 ml Methanol + 450 ml Wasser und 250 ml Methanol + 380 ml Wasser wiederholt. Danach liegt das Diastereomerenpaar A in ca. 96%iger Reinheit vor.
Ausbeute: 1 g (17,2 % der Theorie),
Schmelzpunkt: 234 ° C

| | | | | |
|---|---|---|---|---|
| Ber.: | C 59,26 | H 5,80 | N 7,67 | Cl 9,71 |
| Gef.: | 59,07 | 5,69 | 7,66 | 9,97 |

$^{1}$H-NMR-Spektrum (400 MHz, $CD_3OD/D_6$-DMSO)
δ = 4,949 ppm (dd, >CH-OH)

Diastereomerenpaar B:

3,6 g (0,0095 Mol) eines 1:1-Gemisches der Diastereomerenpaare A und B von N-[2-(4-Carboxymethoxymethoxyphenyl)-1-methylethyl]-2-hydroxy-2-(6-chlor-pyridin-2-yl)ethanamin werden in 20 ml Methanol und 50 ml (0,05 Mol) 1 n Natronlauge bei Raumtemperatur für 45 Minuten gerührt. Die Lösung wird mit 50 ml 1 n Salzsäure versetzt und zur Trockene gebracht. Der Rückstand wird mit 20 ml Methanol und 20 ml Wasser über Nacht gerührt und das ausgefallene Festprodukt abgesaugt. Die Mutterlauge wird eingedampft und der Rückstand mit 25 ml Wasser, dann zweimal mit 50 ml bzw. 30 ml Aceton verrieben und abgesaugt. Danach liegt das Diastereomerenpaar B als Hydrochlorid in 90%iger Reinheit vor.
Ausbeute: 1 g (56 % der Theorie),
Schmelzpunkt: ab 80°C (Zers.)

| | | | | |
|---|---|---|---|---|
| Ber.: | C 53,87 | H 5,52 | N 6,97 | Cl 17,66 |
| Gef.: | 53,82 | 5,66 | 6,86 | 17,50 |

$^{1}$H-NMR-Spektrum (400 MHz, $CD_3OD/D_6$-DMSO)
δ = 4,907 ppm (dd, >CH-OH)

Beispiel 5

N-[2-(4-(2-Hydroxyethoxy)phenyl-1-methylethyl]-2-hydroxy-2-(6-chlor-pyridin-2-yl)ethanamin

Diastereomerenpaar A:

48,6 g eines 1:1-Gemisches der Diastereomerenpaare werden in 500 ml heißem Aceton gelöst und durch Abkühlen im Eisbad zur Kristallisation gebracht, dabei erhält man 15,5 g eines ca. 8:2-Gemisches der Diastereomerenpaare B und A (Schmelzpunkt: 134°C). Das Volumen der Mutterlauge wurde auf 200 ml verringert und über Nacht im Kühlschrank abgekühlt. Das dabei ausgefallene Produkt wurde abgesaugt, in 150 ml heißem Aceton gelöst und durch Abkühlen im Eisbad zur Kristallisation gebracht. Diese Prozedur wurde mit 100 ml Aceton und mit 70 ml Aceton wiederholt, wobei 6 g eines Produktes mit einem Schmelzpunkt von 108°C erhalten wurden. Diese 6 g wurden noch zweimal aus 60 ml bzw. 40 ml Aceton umkristallisiert, wobei das reine Diastereomerenpaar A erhalten wurde.
Ausbeute: 4 g (16,5 % der Theorie),
Schmelzpunkt: 109-110°C

| | | | | |
|---|---|---|---|---|
| Ber.: | C 61,62 | H 6,60 | N 7,98 | Cl 10,10 |
| Gef.: | 61,55 | 6,64 | 7,86 | 10,30 |

$^{1}$H-NMR-Spektrum (400 MHz, $CDCl_3$)
δ = 4,620 ppm (dd, >CH-OH)

Diastereomerenpaar B:

15,5 g eines Diastereomerenpaargemisches (B:A = ca. 8:2) werden viermal aus 600 ml bzw. aus 500 ml, 400 ml und 350 ml Aceton umkristallisiert. Dabei erhält man das Diastereomerenpaar B in 96%iger bis 98%iger Reinheit.
Ausbeute: 7 g (56,5 % der Theorie),
Schmelzpunkt: 142-143°C

| | | | | |
|---|---|---|---|---|
| Ber.: | C 61,62 | H 7,98 | N 7,98 | Cl 10,10 |
| Gef.: | 61,45 | 8,01 | 7,68 | 9,98 |

$^1$H-NMR-Spektrum (400 MHz, $CDCl_3$)
$\delta$ = 4,686 ppm (dd, >C$\underline{H}$-OH)

Beispiel I

Dragée mit 10 mg N-[2-(4-(2-Hydroxyethoxy)phenyl)-1-methylethyl]-2-hydroxy-2-(6-chlor-pyridin-2-yl)-ethanamin

Zusammensetzung:

1 Dragée enthält:

| | |
|---|---|
| (1) Wirksubstanz | 10,0 mg |
| (2) Milchzucker | 69,0 mg |
| (3) Maisstärke | 35,0 mg |
| (4) Polyvinylpyrrolidon | 5,0 mg |
| (5) Magnesiumstearat | 1,0 mg |
| | 120,0 mg |

Herstellung:

(1) + (2) + (3) werden gemischt und mit (4) in einer wäßrigen Lösung befeuchtet. Die feuchte Masse wird durch ein Sieb mit 1,6 mm Maschenweite geschlagen und bei 45 °C im Umlufttrockenschrank getrocknet. Das trockene Granulat wird durch ein Sieb mit 1 mm Maschenweite gegeben und mit (5) gemischt. Die fertige Mischung wird zu Dragéekernen verpreßt.

| | |
|---|---|
| Kerngewicht: | 120,0 mg |
| Durchmesser: | 7,0 mm |
| Wölbungsradius: | 6,0 mm |

Die so hergestellten Dragéekerne werden auf bekannte Weise mit einer Schicht überzogen, die im Wesentlichen aus Zucker und Talkum besteht. Diese Schicht kann auch Farbauszüge enthalten. Die fertigen Dragées werden mit Wachs poliert.

| | |
|---|---|
| Dragéegewicht: | 180,0 mg |

Beispiel II

Dragée mit 50 mg N-[2-(4-(2-Hydroxyethoxy)phenyl)-1-methylethyl]-2-hydroxy-2-(6-chlor-pyridin-2-yl)-ethanamin

Zusammensetzung:

1 Dragée enthält:

| | |
|---|---|
| (1) Wirksubstanz | 50,0 mg |
| (2) Milchzucker | 110,8 mg |
| (3) Maisstärke | 50,0 mg |
| (4) Polyvinylpyrrolidon | 8,0 mg |
| (5) Magnesiumstearat | 1,2 mg |
| | 220,0 mg |

Herstellung:

Die Herstellung erfolgt analog Beispiel I.

| | |
|---|---|
| Kerngewicht: | 220,0 mg |
| Durchmesser: | 9,0 mm |
| Wölbungsradius: | 8,0 mm |
| Dragéegewicht: | 300,0 mg |

Beispiel III

Tabletten mit 150 mg N-[2-(4-(2-Hydroxyethoxy)phenyl)-1-methylethyl]-2-hydroxy-2-(6-chlor-pyridin-2-yl)-ethanamin

Zusammensetzung:

1 Tablette enthält:

| | |
|---|---|
| (1) Wirksubstanz | 150,0 mg |
| (2) Milchzucker | 86,0 mg |
| (3) Maisstärke | 50,8 mg |
| (4) Mikrokristalline Zellulose | 25,0 mg |
| (5) Polyvinylpyrrolidon | 7,0 mg |
| (6) Magnesiumstearat | 1,2 mg |
| | 320,0 mg |

Herstellung:

(1) + (2) + (3) + (4) + (5) werden gemischt und mit Wasser befeuchtet. Die feuchte Masse wird durch ein Sieb mit 1,6 mm Maschenweite geschlagen und bei 45° C getrocknet. Das trockene Granulat wird nochmals durch dasselbe Sieb gegeben und mit (6) gemischt. Aus der fertigen Mischung werden Tabletten gepreßt.

| | |
|---|---|
| Tablettengewicht: | 320,0 mg |
| Durchmesser: | 10,0 mm |

Die Tabletten werden mit einer Teilkerbe versehen, um die Halbierung zu ermöglichen.

Beispiel IV

Hartgelatinekapseln zu 100 mg N-[2-(4-(2-Hydroxyethoxy)phenyl)-1-methylethyl]-2-hydroxy-2-(6-chlor-pyridin-2-yl)ethanamin

Zusammensetzung:

1 Kapsel enthält:
Kapselhülle: Hartgelatinekapseln Größe 3
Kapselinhaltsstoffe:

| | |
|---|---|
| (1) Wirksubstanz | 100,0 mg |
| (2) Lactose x $1H_2O$ | 38,0 mg |
| (3) Maisstärke getrocknet | 60,0 mg |
| (4) Magnesiumstearat | 2,0 mg |
| Kapselfüllgewicht: | 200,0 mg |
| (5) Dest. Wasser | q.s. |

Herstellung:

Ein kleiner Teil Lactose wird ca. 10 %ig in dest. Wasser gelöst (Granulierflüssigkeit). Der Wirkstoff, die restliche Lactose sowie Maisstärke werden gemischt und mit der Granulierflüssigkeit durchfeuchtet. Die Masse wird gesiebt, getrocknet und nach nochmaligem Sieben mit Magnesiumstearat homogen gemischt. Das feinkörnige Granulat wird auf einer geeigneten Maschine in Kapseln abgefüllt.

Beispiel V

Hartgelatinekapseln zu 200 mg N-[2-(4-(2-Hydroxyethoxy)phenyl)-1-methylethyl]-2-hydroxy-2-(6-chlor-pyridin-2-yl)ethanamin

Zusammensetzung:

Kapselhülle: Hartgelatinekapseln Größe 1
Kapselinhaltsstoffe:

| | |
|---|---|
| (1) Wirksubstanz | 200,0 mg |
| (2) Lactose x $1H_2O$ | 47,0 mg |
| (3) Maisstärke getrocknet | 70,0 mg |
| (4) Magnesiumstearat | 3,0 mg |
| Kapselfüllgewicht: | 320,0 mg |
| (5) Dest. Wasser | q.s. |

Herstellung:

Ein kleiner Teil Lactose wird ca. 10 %ig in dest. Wasser gelöst (Granulierflüssigkeit). Der Wirkstoff, die restliche Lactose sowie Maisstärke werden gemischt und mit der Granulierflüssigkeit durchfeuchtet. Die Masse wird gesiebt, getrocknet und nach nochmaligem Sieben mit Magnesiumstearat homogen gemischt. Das feinkörnige Granulat wird auf einer geeigneten Maschine in Kapseln abgefüllt.

**Patentansprüche**

**1.** Pyridylethanolamine der Formel I

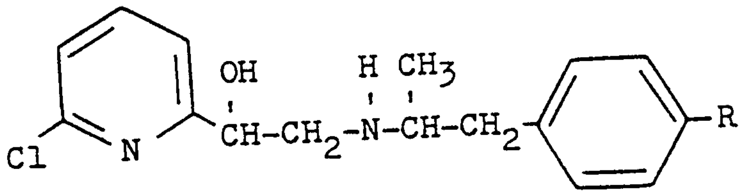 ,(I)

in der
R eine Carboxymethoxy-, Carbomethoxymethoxy- oder 2-Hydroxyethoxygruppe bedeutet, deren optische Isomere, deren Diastereomere und deren Säureadditionssalze.

**2.** N-[2-(4-(2-Hydroxyethoxy)phenyl)-1-methylethyl]-2-hydroxy2-(6-chlor-pyridin-2-yl)ethanamin, dessen optische Isomere und dessen Diastereomere sowie dessen Säureadditionssalze.

**3.** Physiologisch verträgliche Salze der Verbindungen gemäß Anspruch 1 oder 2 mit anorganischen oder organischen Säuren.

**4.** Arzneimittel, enthaltend eine Verbindung gemäß Anspruch 1 oder 2 oder ein physiologisch verträgliches Säureadditionssalz gemäß Anspruch 3 neben gegebenenfalls einem oder mehreren inerten Trägerstoffen und/oder Verdünnungsmitteln.

**5.** Verwendung der Verbindungen nach mindestens einem der Ansprüche 1 bis 3 zur Herstellung eines Arzneimittels, das für die Behandlung des Diabetes mellitus, der Adipositas und zur Behandlung und Prophylaxe atherosklerotischer Veränderungen geeignet ist.

**6.** Verfahren zur Herstellung eines Arzneimittels gemäß Anspruch 4, dadurch gekennzeichnet, daß auf nichtchemischem Wege eine Verbindung nach mindestens einem der Ansprüche 1 bis 3 in einen oder mehrere inerte Trägerstoffe und/oder Verdünnungsmittel eingearbeitet wird.

**7.** Verfahren zur Herstellung der neuen Pyridylethanolamine nach mindestens einem der Ansprüchen 1 bis 3, dadurch gekennzeichnet, daß
a) eine Verbindung der Formel

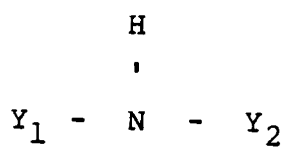 ,(II)

in der $Y_1$ eine Gruppe der Formel

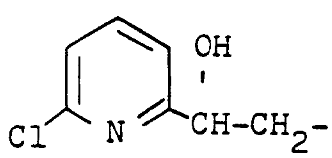

und
$Y_2$ eine gruppe der Formel

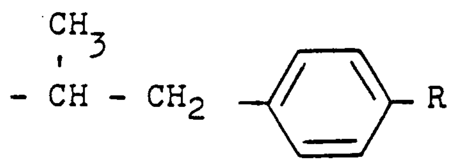

darstellt, in der
R wie in Anspruch 1 oder 2 definiert ist, wobei jedoch einer der Reste $Y_1$ oder $Y_2$ ein Wasserstoffatom darstellen muß mit einer Verbindung der Formel

$Z_1$ - U ,(III)

in der
U eine Gruppe der Formeln

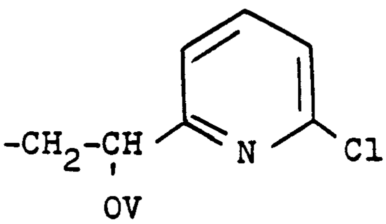

oder

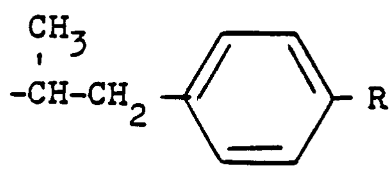

darstellt, wobei
R wie in Anspruch 1 oder 2 definiert ist,
$Z_1$ und V zusammen eine Bindung oder
$Z_1$ eine nukleophile Austrittsgruppe und
V ein Wasserstoffatom bedeuten, umgesetzt wird oder

b) eine Carbonylverbindung der Formel

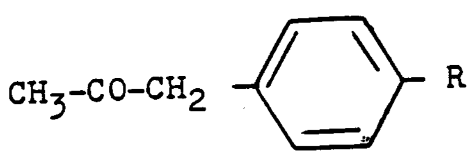

,(IV)

in der
R in Anspruch 1 oder 2 definiert ist, mit einem Amin der Formel

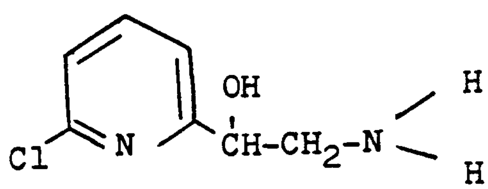

,(V)

reduktiv aminiert wird oder

c) eine Verbindung der Formel

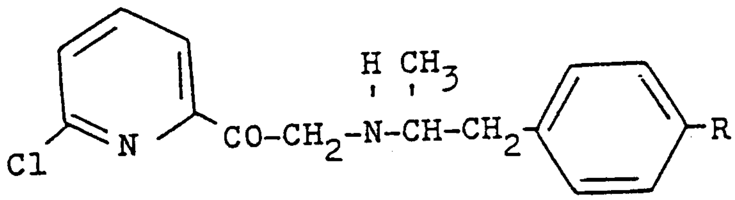

, (VI)

in der
R wie in Anspruch 1 oder 2 definiert ist, reduziert wird oder
d) eine Verbindung der Formel

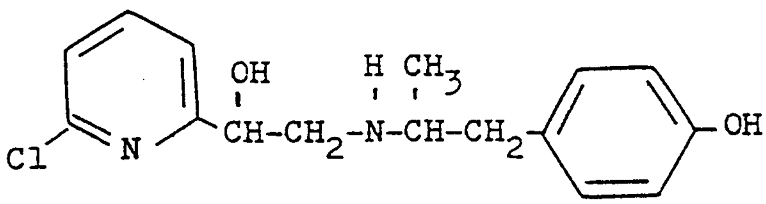

, (VII)

mit einer Verbindung der Formel

$Z_2$ - $R_1$ ,(VIII)

in der
$R_1$ eine Carboxymethyl-, Methoxycarbonylmethyl oder 2-Hydroxy-ethylgruppe und
$Z_2$ eine nukleophile Austrittsgruppe wie ein Halogenatom oder eine Sulfonyloxygruppe oder
$Z_2$ zusammen mit einem β-Wasserstoffatom des Restes $R_1$ ein Sauerstoffatom darstellen, umgesetzt wird,
wobei bei den Umsetzungen a) bis d) reaktionsfähige Gruppen während der Umsetzung durch übliche Schutzreste geschützt sein können, welche nach der Umsetzung durch übliche Verfahren wieder abgespalten werden,
und gewünschtenfalls anschließend eine so erhaltene Verbindung der Formel I, in der R eine Methoxycarbonylmethylgruppe darstellt, mittels Hydrolyse in eine entsprechende Verbindung der Formel I, in der R eine Carboxymethoxygruppe darstellt, übergeführt wird und/oder
eine so erhaltene Verbindung der Formel I in ihre optischen Isomere und Diastereomere aufgetrennt wird und/oder
eine 50 erhaltene Verbindung der Formel I in ihre Säureadditionssalze, insbesondere in ihre physiologisch verträglichen Säureadditionssalze übergeführt werden.

**8.** Futtermittel, enthaltend eine Verbindung gemäß Anspruch I oder 2 oder ein physiologisch verträgliches Säureadditionssalz gemäß Anspruch 3.

## Claims

**1.** Pyridylethanolamines of formula I

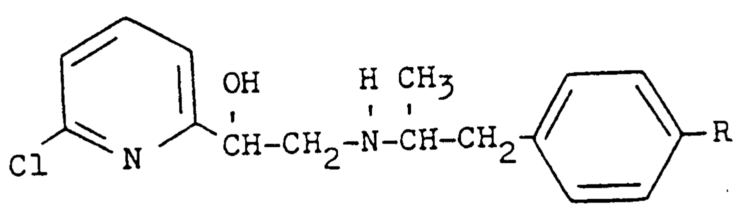

(I)

wherein
R denotes a carboxymethoxy, carbomethoxymethoxy or 2-hydroxyethoxy group, the optical isomers, diastereomers and acid addition salts thereof.

**2.** N-[2-(4-(2-hydroxyethoxy)phenyl)-1-methylethyl]-2-hydroxy-2-(6-chloro-pyridin-2-yl)ethanamine, the optical isomers and diastereomers thereof as well as the acid addition salts thereof.

**3.** Physiologically acceptable salts of the compounds according to claim 1 or 2 with organic or inorganic acids.

**4.** Pharmaceutical compositions containing a compound according to claim 1 or 2 or a physiologically acceptable acid addition salt according to claim 3 optionally together with one or more inert carriers and/or diluents.

**5.** Use of the compounds according to at least one of claims 1 to 3 for preparing a pharmaceutical composition suitable for the treatment of diabetes mellitus and obesity and for the treatment and prevention of atherosclerotic changes.

**6.** Process for preparing a pharmaceutical composition according to claim 4, characterised in that a compound according to at least one of claims 1 to 3 is incorporated in one or more inert carriers and/or diluents by a non-chemical method.

**7.** Process for preparing the new pyridylethanolamines according to at least one of claims 1 to 3, characterised in that
a) a compound of formula

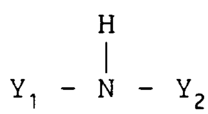

(II)

wherein
$Y_1$ denotes a group of the formula

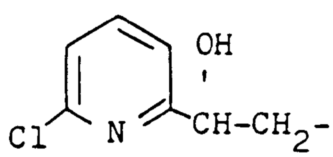

and
$Y_2$ denotes a group of the formula

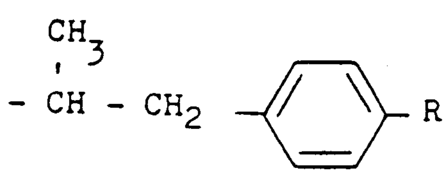

wherein R is defined as in claim 1 or 2, but one of the groups $Y_1$ or $Y_2$ must represent a hydrogen atom,
is reacted with a compound of formula

$Z_1$ - U (III)

wherein
U denotes a group of the formulae

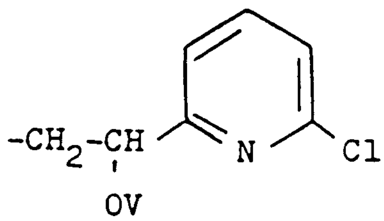

or

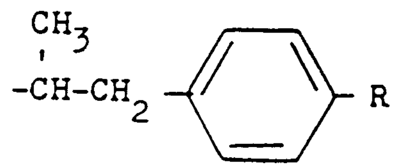

wherein
R is as defined in claim 1 or 2,
$Z_1$ and V together represent a bond or
$Z_1$ denotes a nucleophilic leaving group and
V denotes a hydrogen atom, or
b) a carbonyl compound of formula

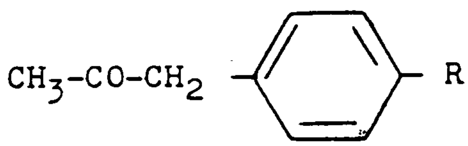

(IV)

wherein
R is defined as in claim 1 or 2, is reductively aminated with an amine of the formula

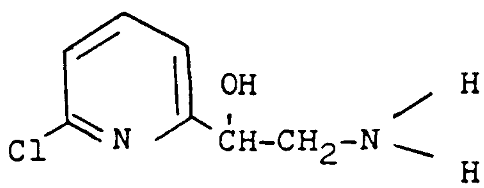

(V)

or
c) a compound of the formula

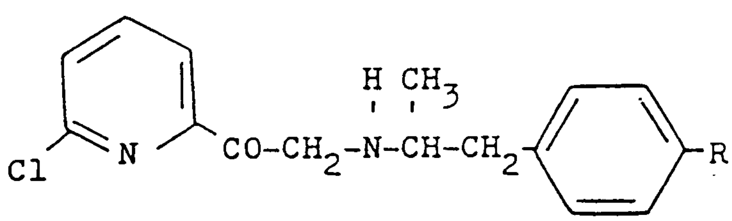

(VI)

wherein
R is defined as in claim 1 or 2, is reduced or
d) a compound of formula

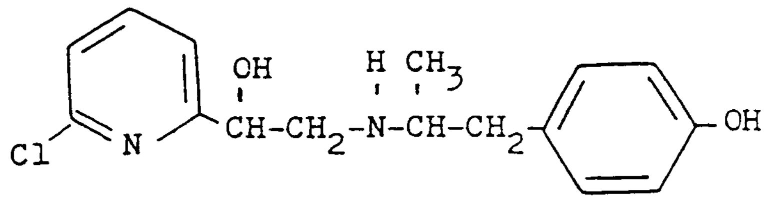

(VII)

is reacted with a compound of formula

$Z_2$ - $R_1$ (VIII)

wherein
$R_1$ denotes a carboxymethyl, methoxycarbonylmethyl or 2-hydroxy-ethyl group and
$Z_2$ denotes a nucleophilic leaving group such as a halogen atom or a sulphonyloxy group or
$Z_2$ together with a $\beta$-hydrogen atom of the group $R_1$ denotes an oxygen atom,
whilst in reactions a) to d) reactive groups may be protected during the reaction by conventional protecting groups which are split off again by conventional methods after the reaction,
and subsequently, if desired, a compound of formula I thus obtained wherein R denotes a methoxycarbonylmethyl group is converted by hydrolysis into a corresponding compound of formula I wherein R denotes a carboxymethoxy group, and/or
a compound of formula I thus obtained is resolved into the optical isomers and diastereomers thereof and/or
a compound of formula I thus obtained is converted into the acid addition salts thereof, more particularly into the physiologically acceptable acid addition salts thereof.

**8.** Feedstuffs containing a compound according to claim 1 or 2 or a physiologically acceptable acid addition salt according to claim 3.

## Revendications

**1.** Pyridyléthanolamines de formule I

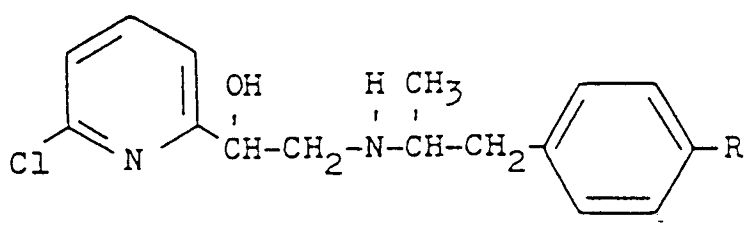

(I)

dans laquelle
R représente un groupe carboxyméthoxy, carbométhoxyméthoxy ou 2-hydroxyéthoxy, leurs isomères optiques, leurs diastéréo-isomères et leurs sels d'addition d'acide.

**2.** N-[2-(4-(2-hydroxyéthoxy)phényl)-l-méthyléthyl]-2-hydroxy-2-(6-chloro-pyridine-2-yl)éthanamine, ses isomères optiques et ses diastéréo-isomères ainsi que ses sels d'addition d'acide.

**3.** Sels acceptables du point de vue physiologique des composés selon la revendication 1 ou 2 avec des acides inorganiques ou organiques.

**4.** Médicament contenant un composé selon la revendication 1 ou 2 ou un sel d'addition d'acide acceptable du point de vue physiologique selon la revendication 3 et éventuellement un ou plusieurs véhicules et/ou diluants inertes.

**5.** Utilisation des composés selon au moins l'une des revendications 1 à 3 pour la fabrication d'un

médicament qui convient pour le traitement du diabète sucré, de l'adiposité et pour le traitement et la prophylaxie des modifications athéroscléreuses.

6. Procédé de fabrication d'un médicament selon la revendication 4, caractérisé en ce qu'un composé selon au moins l'une des revendications 1 à 3 est incorporé par voie non chimique dans un ou plusieurs véhicules et/ou diluants inertes.

7. Procédé de fabrication des nouvelles pyridyléthanolamines selon au moins l'une des revendications 1 à 3, caractérisé en ce que

a) un composé de formule

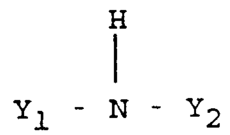

(II)

dans laquelle

$Y_1$ représente un groupe de formule

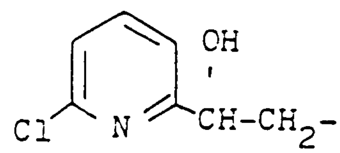

et

$Y_2$ représente un groupe de formule

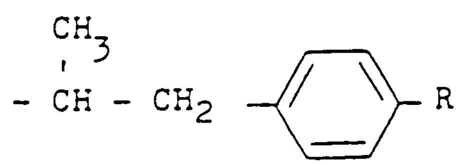

dans laquelle

R est défini comme dans la revendication 1 ou 2, mais dans laquelle cependant l'un des restes $Y_1$ ou $Y_2$ doit représenter un atome d'hydrogène,

est mis à réagir avec un composé de formule

$Z_1$ - U (III)

dans laquelle

U représente un groupe de formule

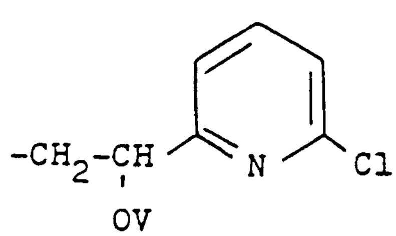

ou

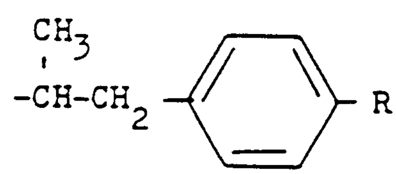

où
R est défini comme dans la revendication 1 ou 2,
$Z_1$ et V représentent ensemble une liaison ou
$Z_1$ représente un groupe partant nucléophile et
V représente un atome d'hydrogène, ou bien
b) un composé carbonylé de formule

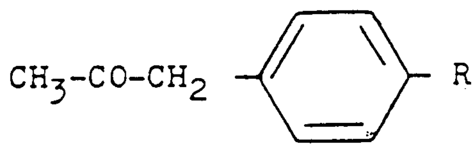

(IV)

dans laquelle
R est défini comme dans la revendication 1 ou 2, est aminé de manière réductrice avec une amine de formule

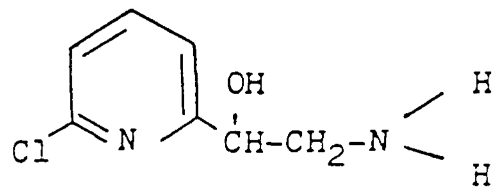

(V)

ou bien
c) un composé de formule

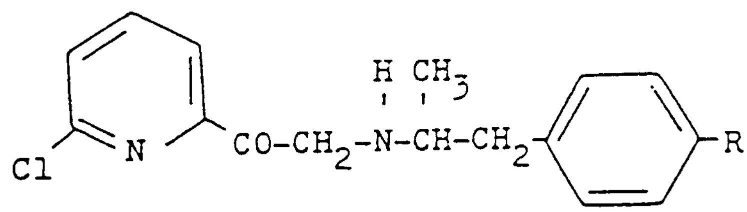

(VI)

dans laquelle
R est défini comme dans la revendication 1 ou 2, est réduit ou bien
d) un composé de formule

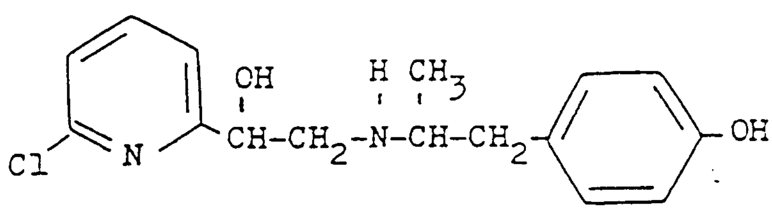

(VII)

est mis à réagir avec un composé de formule

$Z_2 - R_1$ (VIII)

dans laquelle
$R_1$ représente un groupe carboxyméthyle, méthoxycarbonylméthyle ou 2-hydroxyéthyle et

$Z_2$ représente un groupe partant nucléophile tel qu'un atome d'halogène ou un groupe sulfonyloxy ou bien

$Z_2$ représente avec un atome d'hydrogène $\beta$ du reste $R_1$ un atome d'oxygène,

dans les réactions a) à d) les groupes réactifs pouvant être protégés pendant la réaction par des restes protecteurs courants qui sont éliminés de nouveau par des procédés courants après la réaction,

puis, si on le souhaite, un composé de formule I ainsi obtenu dans lequel R représente un groupe méthoxycarbonylméthyle est converti par hydrolyse en un composé correspondant de formule I dans lequel R représente un groupe carboxyméthoxy et/ou

un composé de formule I ainsi obtenu est dédoublé en ses isomères optiques et en ses diastéréo-isomères et/ou

un composé de formule I ainsi obtenu est converti en ses sels d'addition d'acide, en particulier en ses sels d'addition d'acide acceptables du point de vue physiologique.

**8.** Aliments pour animaux contenant un composé selon la revendication 1 ou 2 ou un sel d'addition d'acide acceptable du point de vue physiologique selon la revendication 3.